# EUROPÄISCHE PATENTANMELDUNG

(11) **EP 0 934 751 A1**
(43) Veröffentlichungstag der Anmeldung: **11.08.1999**
(21) Anmeldenummer: 99101979.5
(22) Anmeldetag: 01.02.1999
(51) Int. Cl.: A61M 1/16, A61M 1/36

(54) **Verfahren und Vorrichtung zur Oxygenation von Blut**

(30) Priorität: 05.02.1998 DE 19804536
(71) Anmelder: Institut für Polymerforschung Dresden e.V., 01069 Dresden (DE); Technische Universität Dresden, 01069 Dresden (DE); Albrecht, Detlef Michael Prof.Dr.med., 01277 Dresden (DE)
(72) Erfinder: Jacobasch, Hans-Jörg, 01187 Dresden (DE); Werner, Carsten, 01824 Königstein (DE); Berwald, Siegfried, 01187 Dresden (DE); Körber, Heinz, 01324 Dresden (DE); Albrecht, Detlef Michael, 01277 Dresden (DE); Ragaller, Maximilian, 01307 Dresden (DE); Bleyl, Jörg-Uwe, 01307 Dresden (DE)
(74) Vertreter: Rauschenbach, Marion, Dipl.-Ing.

(57) **Zusammenfassung**

Die Erfindung bezieht sich auf das Gebiet der Medizintechnik und betrifft ein Verfahren und eine Vorrichtung zur Oxygenation von Blut, wie sie z.B. beim klinischen Einsatz im Falle von akutem Lungenversagen (ARDS) eingesetzt werden können.

Die Aufgabe der Erfindung besteht darin, ein Verfahren und eine Vorrichtung für eine langzeitstabile Lungenersatz-Therapie anzugeben.

Gelöst wird die Aufgabe durch ein Verfahren zur Oxygenation von Blut, bei dem eine hydrophobe, flüssige Trägerphase, die die dem Blut zuzuführenden Gase trägt, durch ein Membranmodul (4, 4a, 4b) geleitet wird und der Gasaustausch über eine hydrophile Polymermembran durchgeführt wird.

Die Aufgabe wird weiterhin gelöst durch eine Vorrichtung zur Oxygenation von Blut, bestehend aus einem Membranmodul (4, 4a, 4b), welches zwei Bereiche enthält, die durch mindestens eine hydrophile Polymermembran getrennt sind und wobei in dem einen Bereich ein extrakorporaler Blutkreislauf hindurchgeleitet ist und in dem anderen Bereich eine hydrophobe, flüssige Trägerphase hindurchgeleitet ist.

## Beschreibung

Die Erfindung bezieht sich auf das Gebiet der Medizintechnik und betrifft ein Verfahren und eine Vorrichtung zur Oxygenation von Blut, wie sie z.B. beim klinischen Einsatz im Falle von akutem Lungenversagen (ARDS) eingesetzt werden können. Es handelt sich dabei nicht um ein therapeutisches Verfahren. Das Verfahren ist beispielsweise an Tierblut oder an Blut einer Konserve einsetzbar.

Bisher sind Verfahren und Vorrichtungen zur Blutoxygenation bekannt, die vornehmlich bei herzchirurgischen Eingriffen Anwendung finden. Dabei wird das Blut über einen extrakorporalen Kreislauf durch einen Membranoxygenator gepumpt. Dieser Oxygenator besitzt eine hydrophobe Membran, an deren Gegenseite gasförmiger Sauerstoff strömt (ECMO) [J. Devn Cornish, Robert M Arensman (Hrsg.) 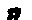 Extracorporeal Life Support", Blackwell Scientific Publications, Boston 1992].

Der Nachteil des genannten Blutoxygenationsverfahrens besteht darin, daß Plasmaproteine in die Membran eindringen und konsekutiv ein Proteindurchbruch in die Gasphase (Plasmaleckage) erfolgt. Dies führt bei Langzeitanwendung zunächst zu einer deutlichen Reduktion der Oxygenierungsleistung und schließlich zum vollständigen Funktionsverlust der Membran. Dieses Problem konnte durch Beschichtung der Membran weitgehend verringert werden (DE 31 06 188).

Der zentrale, inherente Nachteil der mangelnden Biokompatibilität infolge einer großflächigen Wechselwirkungen des Blutes mit einer hydrophoben Oberfläche konnte jedoch hiermit nicht beseitigt werden.

Nach der Veröffentlichung im Annual International Conference of the IEEE Engineering in Medicine and Biology Soc., Vol. 13, No. 4, 1991, S. 1557 - 1559 ist eine Anordnung bekannt, bei der auf der einen Seite einer hydrophilen Membran Blut und auf der anderen Seite der Membran eine Salzlösung strömt. Diese Anordnung eignet sich für die Dialyse. Wegen der geringen Löslichkeit von Sauerstoff in einer Salzlösung läßt sich jedoch auf diese Art und Weise kein ausreichender Oxygenierungseffekt erzielen.

Weiterhin ist ein Verfahren zur Blutoxygenation bekannt, bei dem ein Membrankapillarsystem in die Vena Cava durch einen chirurgisch präparativen Eingriff eingeführt wird. Es wird ebenfalls eine hydrophobe Membran benutzt, an deren Gegenseite gasförmiger Sauerstoff strömt (IVOX) [J.B. Zwischenberger, ASAIO Journal 42:3 (1996) 207]. Dieses Verfahren hat sich wegen zu häufiger Komplikationen nicht in der Praxis durchgesetzt.

Die Aufgabe der Erfindung besteht darin, ein Verfahren und eine Vorrichtung zur Oxygenation von Blut anzugeben, durch die eine langzeitstabile Lungenersatz-Therapie ermöglicht wird.

Die Aufgabe wird durch die in den Ansprüchen 1 und 4 angegebene Erfindung gelöst. Weiterbildungen sind Gegenstand der Unteransprüche.

Bei der erfindungsgemäßen Vorrichtung werden hydrophile, biokompatible Membranen in Verbindung mit hydrophoben, flüssigen Trägermedien eingesetzt, durch die das erfindungsgemäße Verfahren realisiert wird.

Bekanntermaßen wird Perfluorcarbon als Trägerflüssigkeit für Sauerstoff in Vorrichtungen nach dem Stand der Technik bereits eingesetzt. Hierbei wird mit Sauerstoff gesättigtes Perfluorcarbon direkt in die Blutbahn gegeben. Bei diesem Verfahren kommt es jedoch zu einer unkontrollierten Anreicherung von Perfluorcarbon im Organismus, was eine Einführung in die Praxis verhindert hat. Bekannt ist auch, daß eine Flüssigkeit mit einer so geringen Oberflächenspannung, wie das Perfluorcarbon, durch eine hydrophile Membran hindurchtritt und damit eine Barrierewirkung nicht erreichbar ist.

Demgegenüber wird bei dem erfindungsgemäßen Verfahren eine hydrophile Membran eingesetzt, bei der an deren einen Seite ein Blutstrom fließt. Danach wird auf der anderen Membranseite der Perfluorcarbonkreis in Betrieb genommen. Überraschenderweise tritt nun kein Perfluorcarbon auf die Blutseite über, auch wenn auf der Perfluorcarbonseite der Druck deutlich über dem Druck auf der Blutseite liegt.

Der Aufbau der erfindungsgemäßen Vorrichtung besteht aus einem Membranmodul mit mindestens einer hydrophilen Membran. Diese Membran ist vorzugsweise eine Kapillarmembran mit 50 bis 250 µm Innendurchmesser und einer Wandstärke von 3 bis 15 µm. Diese Kapillarmembran kann in Form eines Faserbündels angeordnet sein mit im wesentlichen parallel zueinander verlaufenden Kapillaren. Vorteilhaft ist es jedoch, diese Kapillarmembran in Form sich überkreuzender Lagen zu wickeln, so daß der Außenraum um die Kapillaren aus einer Vielzahl von Erweiterungen und Verengungen besteht. Durch die geometrischen Verhältnisse der Kapillarmembran (Durchmesser zu Wandstärke) sind Innenoberfläche und Außenoberfläche annähernd gleich groß. Der Gastransport kann sowohl von innen nach außen als auch von außen nach innen erfolgen. Vorzugsweise strömt das flüssige, hydrophobe Trägermedium durch den Innenraum der Kapillaren und der Blutstrom wird durch den Außenraum geführt.
Das Trägermedium wird durch das Membranmodul mit einer Verdrängerpumpe, vorzugsweise mit einer drehzahl-gesteuerten Zahnradpumpe, gepumpt Die Anreicherung mit Sauerstoff wird durch Kontakt der freien Flüssigkeitsoberfläche mit gasförmigem Sauerstoff erreicht. Dazu wird die Trägerflüssigkeit über ein Begasungsgefäß mit Füllkörperschüttung, die vorzugsweise aus hohlzylinderförmige Glaskörpern (Außendurchmesser 7 bis 10 mm, Länge 7 bis 10 mm, Wandstärke 1 bis 2 mm) besteht, geleitet. Der gasförmige Sauerstoff strömt im Gegenstrom. Im Begasungsgefäß wird über Drosselung in der Abgasleitung ein Druck im Bereich zwischen 2 kPa und 50 kPa eingestellt.
Der Blutstrom wird mit einer drehzahl-gesteuerten Peristaltik-Schlauchpumpe wie bei der bekannten ECMO erzeugt.
Die Volumenströme müssen entsprechend dem Bedarf des Organismus eingestellt werden, wobei der Volumenstrom des Trägermediums im Verhältnis zum Blutstrom, vorzugsweise in einem Bereich von 0,8 zu 1 bis 1,5 zu 1, eingestellt wird.

Die Erfindung wird an zwei Ausführungsbeispielen näher erläutert:

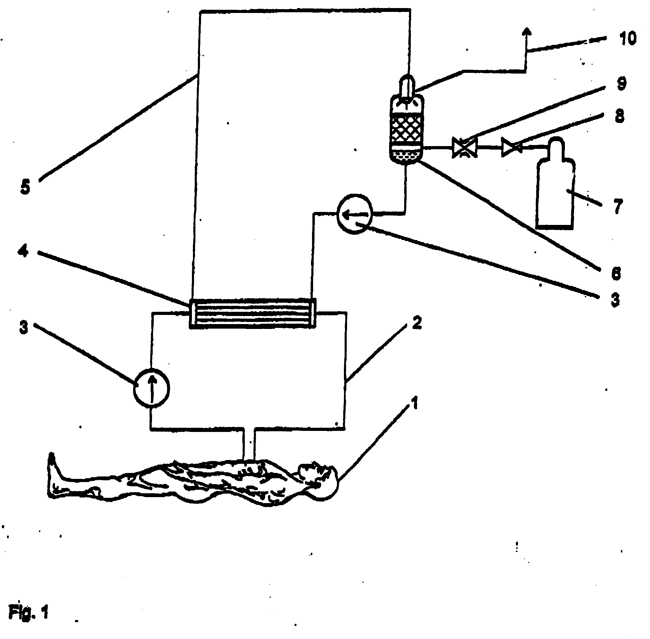

Dabei zeigt
Fig. 1 den Aufbau der erfindungsgemäßen Vorrichtung mit einem Trägermedienkreis
   und

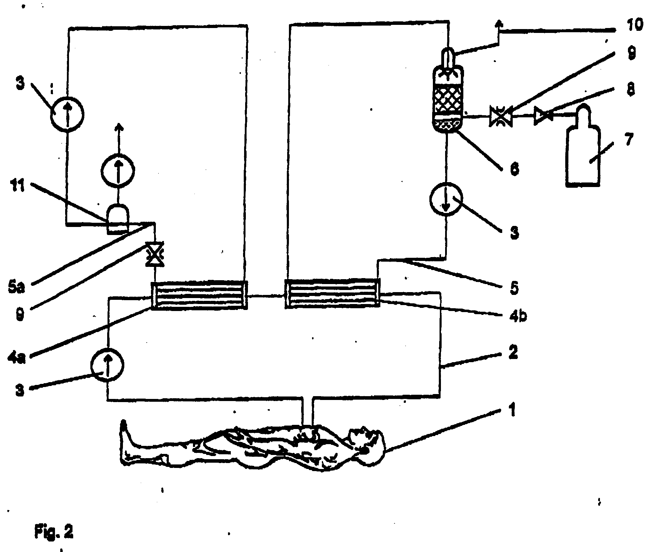

Fig. 2 den Aufbau der erfindungsgemäßen Vorrichtung mit zwei Trägermedienkreisen

### Beispiel 1

An einem Patient 1 ist ein Blutkreisbypass 2 gelegt, durch den mit einer Pumpe 3 ein Blutstrom über einen Membranmodul 4 mit hydrophiler Membran gepumpt wird. An der Gegenseite der Membran ist ein Perfluorcarbonkreis 5 angeschlossen, der über eine Pumpe 3 umgewälzt wird.
Der Gasaustausch erfolgt an einem Membranmodul 4, der mit einer hydrophilen Membran ausgerüstet ist.
Die Trägerflüssigkeit, Perfluorcarbon, durchströmt das Begasungsgefäß 6, wo sie über eine Austauschfläche, eine Füllkörperschüttung, direkt mit gasförmigem Sauerstoff in Kontakt gebracht wird.
Der Sauerstoff strömt an der Austauschfläche im Gegenstrom zu der Trägerflüssigkeit und wird in bekannter Weise aus einer Druckflasche 7 über einen Druckminderer 8 und ein Dosierventil 9 entnommen.

Die Trägerflüssigkeit sättigt sich beim Durchströmen der Begasungseinheit 6 mit Sauerstoff auf, wobei andere gelöste Gase z.B. Kohlendioxid an der Austauschfläche entweichen.
Die mit Sauerstoff gesättigte Trägerflüssigkeit wird mit einer Zahnradpumpe 3 durch den Membranmodul 4 gepumpt. Dabei wird über die hydrophile Membran Sauerstoff an das Blut übertragen und Kohlendioxid vom Blut an die Trägerflüssigkeit überführt. Überraschenderweise tritt über die hydrophile Membran auch bei langen Kontaktzeiten kein Blutplasma an die Trägerflüssigkeit, das Perfluorcarbon, über, und ebenso gelangt keine Trägerflüssigkeit in das Blut.

### Beispiel 2

An einem Patient 1 ist ein Blutkreisbypass 2 gelegt, durch den mit einer Pumpe 3 ein Blutstrom über einen Entgasungs-Membranmodul 4a und einen Oxygenierungs-Membranmodul 4b gepumpt wird.
Für den Gasaustausch mit dem Blut werden zwei Membranmodule (4a, 4b) verwendet.
Der Entgasungs-Membranmodul 4a wird mit einer zuvor entgasten Trägerflüssigkeit, Perfluorcarbon, beschickt und entfernt Kohlendioxid aus dem Blut. Das dafür verwendete Perfluorcarbon wird permanent über ein Dosierventil 9 in einem Entgasungsgefäß 11 durch Unterdruck entgast, mit einer Zahnradpumpe 3 angesaugt und mit ausreichendem Überdruck durch den Entgasungs-Membranmodul 4a gepumpt.
An der Gegenseite des Oxygenierungs-Membranmodul 4b ist ein Perfluorcarbonkreis 5 angeschlossen, der über eine Pumpe 3 umgewälzt wird, wobei das Perfluorcarbon über ein Begasungsgefäß 7 mit Sauerstoff aufgesättigt wird. Der Sauerstoff wird einer Druckgasflasche 7 über einen Druckminderer 8 und ein Dosierventil 9 entnommen, über das Begasungsgefäß 6 geleitet und über die Abgasleitung 10 in die Atmosphäre geblasen.

### Bezugszeichenliste

- 1: - Patient
- 2: - Blutkreisbypass
- 3: - Pumpe
- 4: - Membranmodul
- 4a: - Entgasungs-Membranmodul
- 4b: - Oxygenierungs-Membranmodul
- 5: - Perfluorcarbonkreis
- 5a: - Entgasungs-Perfluorcarbonkreis
- 6: - Begasungsgefäß
- 7: - Druckgasflasche
- 8: - Druckminderer
- 9: - Dosierventil
- 10: - Abgasleitung
- 11: - Entgasungsgefäß

## Patentansprüche

1. Verfahren zur Oxygenation von Blut, bei dem eine hydrophobe, flüssige Trägerphase, die die dem Blut zuzuführenden Gase trägt, durch ein Membranmodul (4, 4a, 4b) geleitet wird und der Gasaustausch über eine hydrophile Polymermembran durchgeführt wird und die Trägerphase mit den aus dem Blut zu entfernenden Gasen aus dem Membranmodul (4, 4a, 4b) in eine Begasungseinheit (6) geleitet wird, in der die aus dem Blut zu entfernenden Gase an einer Austauschfläche entfernt und die hydrophobe, flüssige Trägerphase mit den dem Blut zuzuführenden Gase beaufschlagt wird.

2. Verfahren nach Anspruch 1, bei dem als hydrophile Polymermembran eine Hämodialysemembran eingesetzt wird.

3. Verfahren nach Anspruch 1, bei dem als Trägerphase hydrophobe, flüssige Perfluorcarbone eingesetzt werden.

4. Vorrichtung zur Oxygenation von Blut, bestehend aus einem Membranmodul (4, 4a, 4b), welches zwei Bereiche enthält, die durch mindestens eine hydrophile Polymermembran getrennt sind und wobei in dem einen Bereich ein extrakorporaler Blutkreislauf hindurchgeleitet ist und in dem anderen Bereich eine hydrophobe, flüssige Trägerphase hindurchgeleitet ist, und weiter bestehend aus einer Begasungseinheit (6), der die dem Blut zuzuführende Gase zugeleitet sind.

5. Vorrichtung nach Anspruch 4, bei der als hydrophile Polymermembran eine Hämodialysemembran eingesetzt ist.

6. Vorrichtung nach Anspruch 4, bei der als Trägerphase hydrophobe, flüssige Perfluorcarbone eingesetzt sind.
